# EUROPEAN PATENT APPLICATION

(11) **EP 3 777 954 A1**
(43) Date of publication of application: **17.02.2021**
(21) Application number: 19776485.5
(22) Date of filing: 25.03.2019
(51) Int. Cl.: A61M 37/00

(54) **MOLD FOR MANUFACTURING TRANSDERMAL ABSORPTION SHEET, AND DEVICE AND METHOD FOR MANUFACTURING TRANSDERMAL ABSORPTION SHEET HAVING NEEDLE-LIKE PROTRUSION**

(30) Priority: 30.03.2018 JP 2018068100
(71) Applicant: FUJIFILM CORPORATION, Minato-ku Tokyo 106-8620 (JP)
(72) Inventor: USA, Toshihiro, Ashigarakami-gun, Kanagawa 258-8538 (JP); CHAI, Satoshi, Ashigarakami-gun, Kanagawa 258-8538 (JP)
(74) Representative: Klunker IP Patentanwälte PartG mbB
(86) International application number: PCT/JP2019/012409
(87) International publication number: WO 2019/188932

(57) **Abstract**

Provided are a mold for manufacturing a percutaneous absorption sheet that can be detected from an image, and an apparatus and a method for manufacturing a percutaneous absorption sheet having a needle-like protrusion using the mold. The mold is a mold for manufacturing a percutaneous absorption sheet in which a plurality of needle-like recessed portions are disposed, and the problem is solved by a mold for manufacturing a percutaneous absorption sheet in which the mold has a gray color in which a brightness value in a case where a brightness in an HSL (Hue Saturation Lightness) color space is represented in 256 levels is in a range of 30 or more and 200 or less.

## Description

### BACKGROUND OF THE INVENTION

### 1. Field of the Invention

The present invention relates to a mold for manufacturing a percutaneous absorption sheet, and an apparatus and a method for manufacturing a percutaneous absorption sheet having a needle-like protrusion, and particularly to an apparatus and a method for manufacturing a percutaneous absorption sheet having a needle-like protrusion by ejecting a drug solution from a drug solution ejection nozzle toward a needle-like recessed portion of a mold, and a mold used for manufacturing the same.

### 2. Description of the Related Art

In recent years, as a novel dosage form capable of injecting drugs such as insulin, vaccines, and human growth hormone (hGH) into the skin without pain, a microneedle array has been known. The microneedle array is an array of microneedles (also referred to as fine needles, or small needles) which contain drugs and are biodegradable. By attaching this microneedle array to the skin, each microneedle pierces the skin, and these microneedles are absorbed in the skin such that the drugs contained in each microneedle can be administered into the skin. Microneedle arrays are also called percutaneous absorption sheets.

In order to produce a molded product having a fine protruding pattern of a percutaneous absorption sheet, a resin mold having an inverted shape is formed from a plate precursor having the fine protruding pattern, and a molded product is produced from the mold. There is a demand for improving the productivity of molded products having such fine patterns, and various proposals have been made.

In particular, in a step of filling a drug solution, since the filling amount thereof is related to the drug dose, it is necessary to reliably fill the mold with a very small amount of the drug solution in a constant amount with high accuracy.

In JP2016-112169A, a technique is described in which a mold (corresponding to a mold) mounted on an XYZ stage is moved relative to a nozzle of a liquid droplet ejecting device, a liquid droplet ejected from the nozzle is caused to directly land on each of recessed portions of the mold, and each of the recessed portions is filled with the liquid droplet. According to this technique, the filling amount of the drug solution can be adjusted with high accuracy.

### SUMMARY OF THE INVENTION

As in the technique described in JP2016-112169A, in order to cause the liquid droplets ejected from the nozzles to land on the recessed portions of the mold, positioning between the position of the nozzles and the position of the mold with high accuracy is important. For this reason, it is necessary to detect the mold from an image obtained by imaging the mold, but JP2016-112169A does not disclose the details of the positioning.

The present invention has been made taking the above circumstances into consideration, and an object thereof is to provide a mold for manufacturing a percutaneous absorption sheet that can be detected from an image, and an apparatus and a method for manufacturing a percutaneous absorption sheet having a needle-like protrusion using the mold.

In order to achieve the above object, according to an aspect of the present invention, there is provided a mold for manufacturing a percutaneous absorption sheet in which a plurality of needle-like recessed portions are disposed, in which the mold has a gray color in which a brightness value in a case where a brightness in an HSL (Hue Saturation Lightness) color space is represented in 256 levels is in a range of 30 or more and 200 or less.

According to the aspect, the mold can be detected from an image obtained by imaging the mold.

It is preferable that the mold has a gray color in which the brightness value is in a range of 75 or more. Accordingly, the mold can be detected from the image obtained by imaging the mold.

It is preferable that the mold has a gray color in which the brightness value is in a range of 150 or less. Accordingly, the mold can be detected from the image obtained by imaging the mold.

It is preferable that the mold has a gray color in which a saturation value in a case where a saturation in an HSL color space is represented in 256 levels is in a range of 0 or more and 25 or less. Accordingly, the mold can be detected from the image obtained by imaging the mold.

It is preferable that the mold includes a transparent resin, a white colorant, and a black colorant. Accordingly, the mold having a gray brightness value can be produced using the transparent resin.

It is preferable that a mass ratio between the white colorant and the black colorant is 3:1 to 10:1. Accordingly, the brightness value of the gray color of the mold can be appropriately produced.

It is preferable that the mass ratio between the white colorant and the black colorant is 7:1. Accordingly, the brightness value of the gray color of the mold can be appropriately produced.

It is preferable that an amount of the white colorant and the black colorant is 5 mass% or less. Accordingly, the mold can be appropriately produced.

It is preferable that the resin is a silicone resin. Accordingly, the mold can be appropriately produced.

In order to achieve the above object, according to another aspect of the present invention, there is provided a method for manufacturing a percutaneous absorption sheet having a needle-like protrusion, the method comprising: an image detection step of detecting the mold for manufacturing a percutaneous absorption sheet from an image obtained by imaging the mold for manufacturing a percutaneous absorption sheet; a positioning step of mechanically positioning at least one of a drug solution ejection nozzle or the mold for manufacturing a percutaneous absorption sheet based on a detection result of the image detection step; and an ejection step of ejecting a drug solution from the drug solution ejection nozzle toward a needle-like recessed portion of the mold for manufacturing a percutaneous absorption sheet.

According to the aspect, since the mold can be detected from the image obtained by imaging the mold, the mold can be appropriately positioned and the drug solution can be appropriately supplied to the needle-like recessed portion.

It is preferable that a suction step of suctioning the mold for manufacturing a percutaneous absorption sheet to fill the needle-like recessed portion with the drug solution is further included. Accordingly, the needle-like recessed portion can be appropriately filled with the drug solution.

It is preferable that the mold for manufacturing a percutaneous absorption sheet is placed on a transporting jig, and the transporting jig is provided with an adsorption hole for suctioning the mold for manufacturing a percutaneous absorption sheet in the suction step. Accordingly, the mold can be properly handled, and the needle-like recessed portion can be appropriately filled with the drug solution.

It is preferable that a drying step of drying the drug solution filling the needle-like recessed portion is further comprised. Accordingly, the percutaneous absorption sheet can be appropriately manufactured.

In order to achieve the above object, according to another aspect of the present invention, there is provided an apparatus for manufacturing a percutaneous absorption sheet having a needle-like protrusion, the apparatus comprising: a camera that images the mold for manufacturing a percutaneous absorption sheet; an image detector that detects the mold for manufacturing a percutaneous absorption sheet from an image obtained by imaging the mold; a drug solution ejection nozzle that ejects a drug solution toward a needle-like recessed portion of the mold for manufacturing a percutaneous absorption sheet; and a positioning unit that mechanically positions at least one of the drug solution ejection nozzle or the mold for manufacturing a percutaneous absorption sheet based on a detection result of the image detector.

According to the aspect, since the mold can be detected from the image obtained by imaging the mold, the mold can be appropriately positioned and the drug solution can be appropriately supplied to the needle-like recessed portion.

According to the aspects of the present invention, the mold can be detected from an image obtained by imaging the mold. Accordingly, the drug solution can be appropriately supplied to the needle-like recessed portion of the mold.

### BRIEF DESCRIPTION OF THE DRAWINGS

Fig. 1 is a perspective view illustrating an example of a percutaneous absorption sheet.
Fig. 2 is a perspective view illustrating an example of a mold.
Fig. 3 is a partially enlarged view of a section 3-3 in Fig. 2.
Fig. 4 is a process diagram illustrating a method for producing a mold by injection molding.
Fig. 5 is a process diagram illustrating the method for producing a mold by injection molding.
Fig. 6 is a process diagram illustrating the method for producing a mold by injection molding.
Fig. 7 is a process diagram illustrating the method for producing a mold by injection molding.
Fig. 8 is a process diagram illustrating the method for producing a mold by injection molding.
Fig. 9 is a process diagram illustrating the method for producing a mold by injection molding.
Fig. 10 is a process diagram illustrating the method for producing a mold by injection molding.
Fig. 11 is a process diagram illustrating the method for producing a mold by injection molding.
Fig. 12 is a process diagram illustrating the method for producing a mold by injection molding.
Fig. 13 is a perspective view of a transporting jig on which a mold is mounted.
Fig. 14 is a flowchart showing each step of a method for manufacturing a percutaneous absorption sheet.
Fig. 15 is a schematic configuration diagram of a drug solution filling apparatus used in a drug solution filling step.
Fig. 16 is a block diagram illustrating an electrical configuration of the drug solution filling apparatus.
Fig. 17 is a flowchart showing each step included in the drug solution filling step.
Fig. 18 is a view showing a taken image of each sample, a histogram of a brightness of the taken image, and whether or not image recognition is possible.
Fig. 19 is a view showing a taken image of each sample, a histogram of a brightness of the taken image, and whether or not image recognition is possible.
Fig. 20 is an example of an 8-bit grayscale sample.
Fig. 21 is a diagram showing results of evaluation of a gray color of a mold.

### DESCRIPTION OF THE PREFERRED EMBODIMENTS

Hereinafter, preferred embodiments of the present invention will be described with reference to the accompanying drawings. The present invention is described by the following preferred embodiments. Modifications can be made by various methods without departing from the scope of the present invention, and other embodiments than the present embodiment can also be used. Therefore, all modifications within the scope of the present invention are included in the appended claims.

Here, in the figures, like elements having similar functions are denoted by like reference numerals. In addition, in this specification, in a case where a numerical value range is expressed using "to", the numerical value range includes the numerical values of the upper limit and the lower limit indicated by "to".

### <Configuration of Percutaneous Absorption Sheet>

First, an example of a percutaneous absorption sheet manufactured by a method for manufacturing a percutaneous absorption sheet of the present embodiment will be described.

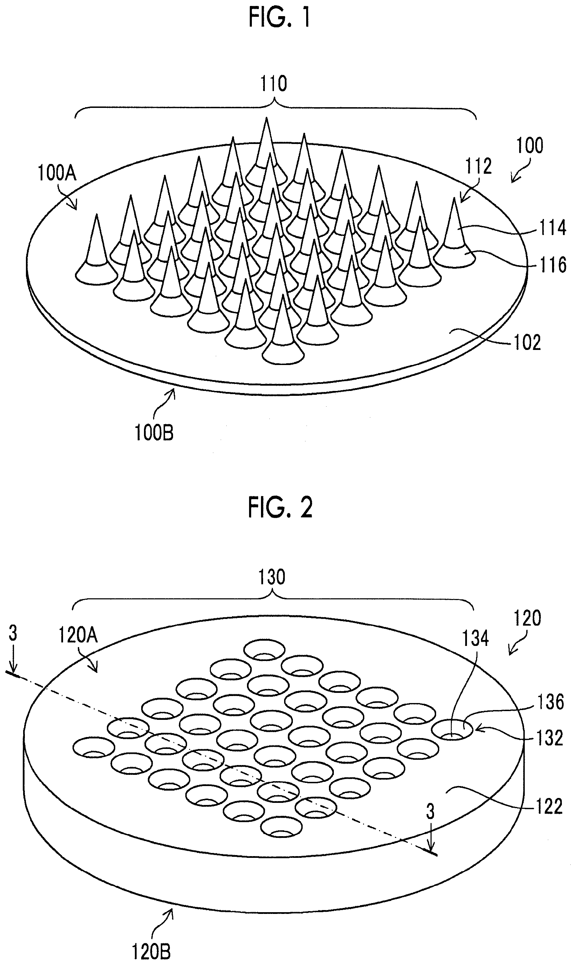

Fig. 1 is a perspective view illustrating an example of a percutaneous absorption sheet 100. The percutaneous absorption sheet 100 has a front surface 100A and a rear surface 100B, and is constituted by a sheet-like sheet portion 102 and a protruding pattern 110.

"Sheet-like" means a thin flat shape as a whole with respect to the two opposing front and rear surfaces 100A and 100B having a large area, and the front and rear surfaces 100A and 100B do not need to be completely flat. Although the sheet portion 102 illustrated in Fig. 1 is circular in a plan view, the sheet portion 102 may be rectangular, polygonal, elliptical, or the like.

The protruding pattern 110 has a plurality of needle-like protrusions 112. The needle-like protrusions 112 are provided on the front surface 100A. The needle-like protrusion 112 includes a needle portion 114, and a frustum portion 116 that connects the needle portion 114 to the sheet portion 102.

A plurality of the frustum portions 116 are formed on the front surface 100A of the percutaneous absorption sheet 100. The frustum portion 116 has two bottom surfaces and has a three-dimensional structure surrounded by a conical surface. The bottom surface (lower bottom surface) of the two bottom surfaces of the frustum portion 116 having a large area is connected to the sheet portion 102. The bottom surface (upper bottom surface) of the two bottom surfaces of the frustum portion 116 having a small area is connected to the needle portion 114. That is, of the two bottom surfaces of the frustum portion 116, the area of the bottom surface in a direction away from the sheet portion 102 is small.

The needle portion 114 has a bottom surface with a large area and a shape having a narrowest area at the distal end apart from the bottom surface. Since the bottom surface of the needle portion 114 having a large area is connected to the upper bottom surface of the frustum portion 116, the needle portion 114 has a tapered shape in a direction away from the frustum portion 116. Therefore, the needle-like protrusion 112 constituted by the needle portion 114 and the frustum portion 116 has a tapered shape as a whole from the sheet portion 102 toward the distal end. A plurality of, for example, 4 to 2500 needle-like protrusions 112 are provided on the sheet portion 102. However, the number thereof is not limited thereto.

In Fig. 1, the frustum portion 116 has a truncated cone shape, and the needle portion 114 has a cone shape. Depending on the degree of insertion of the needle portion 114 into the skin, the shape of the distal end of the needle portion 114 can be appropriately changed to a curved surface having a radius of curvature of 0.01 µm or more and 50 µm or less, a flat surface, or the like.

### <Configuration of Mold>

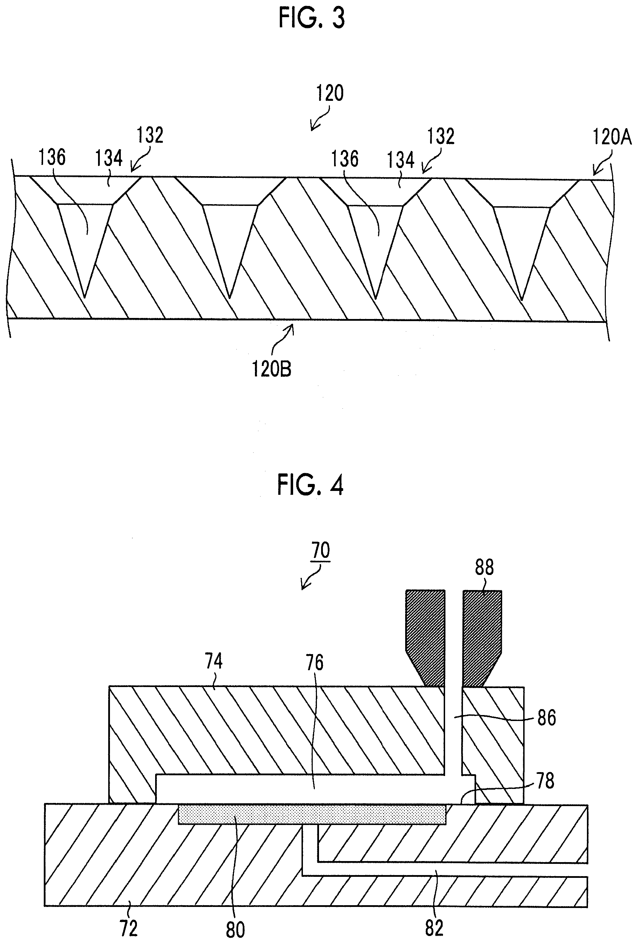

Fig. 2 is a perspective view illustrating an example of a mold 120 (mold for manufacturing a percutaneous absorption sheet) for manufacturing the percutaneous absorption sheet 100. In addition, Fig. 3 is a partially enlarged view of a section 3-3 in Fig. 2. The mold 120 has a front surface 120A and a rear surface 120B, and is constituted by a flat portion 122 and a recessed pattern 130.

The flat portion 122 has a flat shape corresponding to the sheet portion 102 of the percutaneous absorption sheet 100. The recessed pattern 130 is constituted by a plurality of needle-like recessed portions 132. The needle-like recessed portion 132 has a shape corresponding to the needle-like protrusion 112 of the percutaneous absorption sheet 100, and is constituted by a distal end recessed portion 134 corresponding to the needle portion 114 and a cup portion 136 corresponding to the frustum portion 116.

The distal end recessed portion 134 has a tapered shape in a depth direction of the mold 120. The distal end recessed portion 134 can have a diameter of 150 µm to 500 µm and a height of 150 µm to 2000 µm. The cup portion 136 has a shape that narrows in the depth direction of the mold 120. The cup portion 136 can have a diameter of 500 µm to 1000 µm and a height of 100 µm to 500 µm.

The shape of the needle-like recessed portion 132 is not limited to this example. A rocket shape provided with an intermediate recessed portion having a constant width in the depth direction, such as a cylinder, a quadrangular prism, or a polygonal column, between the distal end recessed portion 134 and the cup portion 136 may be applied. In addition, a through-hole that reaches the rear surface 120B and penetrates the mold 120 may be formed at the distal end of the tapered shape. The arrangement, pitch, number, and the like of the needle-like recessed portions 132 are determined based on the arrangement, pitch, number, and the like of the needle-like protrusions 112 necessary for the percutaneous absorption sheet 100.

### <Method for Producing Mold>

A method for producing a mold by injection molding will be described with reference to process diagrams of Figs. 4 to 12.

As illustrated in Fig. 4, a mold 70 including a first mold 72 and a second mold 74 is prepared. By clamping the first mold 72 and the second mold 74, a cavity 76 is formed inside the mold 70. The cavity 76 means a space filled with a resin.

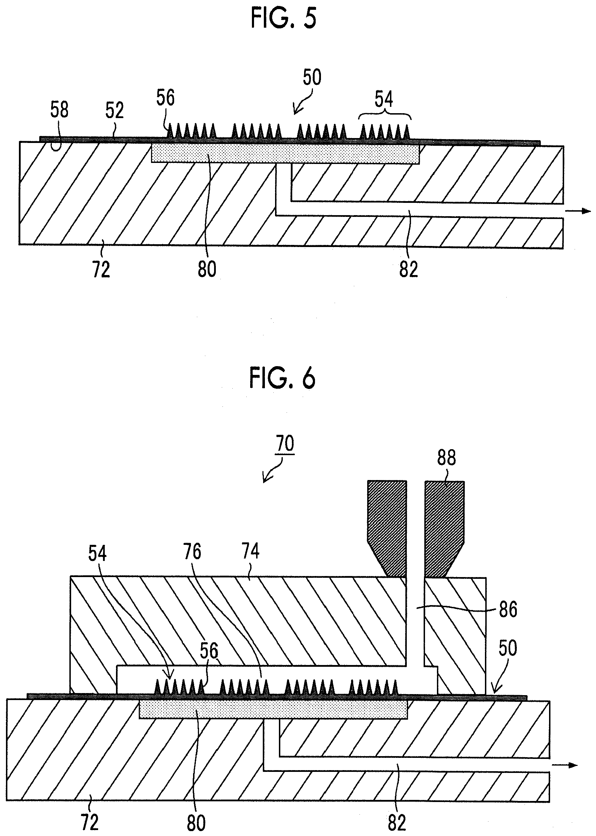

In addition, as illustrated in Fig. 5, an electroform 50 is prepared. On a first surface 52 of the electroform 50, a protruding pattern 54 which is an inverted shape of a mold to be produced is formed. The protruding pattern 54 is a state in which a plurality of needle-like protrusions 56 are arranged in an array. The needle-like protrusions 56 are produced according to the shape of the mold to be produced.

Since the electroform 50 is fixed to the first mold 72, the side to which the electroform 50 is fixed is formed of the flat surface 78. The first mold 72 comprises an adsorption plate 80 on the flat surface 78 as a device for fixing the electroform 50. The first mold 72 comprises a suction pipe 82 in which gas communicates with the adsorption plate 80. The suction pipe 82 is connected to a vacuum pump (not illustrated). By driving the vacuum pump, air can be suctioned from the front surface of the adsorption plate 80. For example, the adsorption plate 80 is formed of a porous member. Examples of the porous member include a metal sintered body, a resin, and a ceramic.

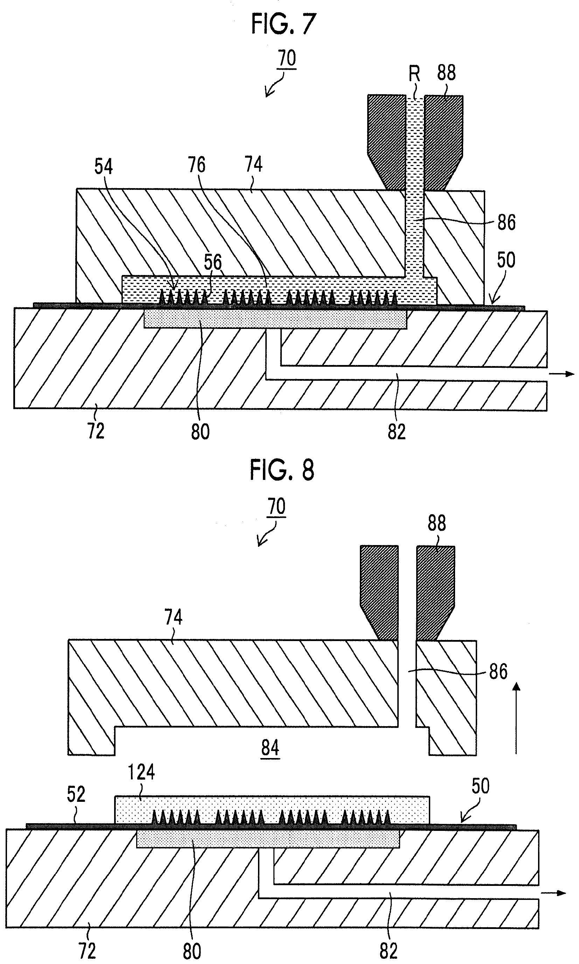

A depression 84 is formed on the cavity 76 side of the second mold 74. In the present embodiment, the cavity 76 is formed by the flat surface 78 of the first mold 72 and the depression 84 (see Fig. 8) of the second mold 74. By configuring the first mold 72 and the second mold 74 as described above, releasing of the mold is facilitated as described later.

A gate 86 that communicates with the cavity 76 is formed in the second mold 74. The gate 86 serves as an injection port for a resin into the cavity 76 of the mold 70. The gate 86 communicates with an injection molding machine 88 that supplies the resin into the mold 70.

As illustrated in Fig. 5, the first mold 72 and the second mold 74 are opened, and the electroform 50 having the protruding patterns 54 is placed on the first mold 72. By suctioning air using the vacuum pump via the suction pipe 82, the second surface 58 of the electroform 50 is vacuum-adsorbed onto the adsorption plate 80.

In the present embodiment, the case where the electroform 50 is fixed to the first mold 72 by vacuum adsorption is illustrated, but the present embodiment is not limited thereto. For example, instead of the adsorption plate 80, a magnet may be provided in the first mold 72 to fix the electroform 50 to the first mold 72 by utilizing the magnetic force. Therefore, it is preferable to fix the electroform 50 to the first mold 72 by at least one of vacuum adsorption or magnetic force.

As illustrated in Fig. 6, in order to form the cavity 76, the first mold 72 and the second mold 74 are clamped. During the clamping, the electroform 50 is clamped by the first mold 72 and the second mold 74.

As illustrated in Fig. 7, the resin R is supplied from the injection molding machine 88 to the cavity 76 via the gate 86. The resin R fills the cavity 76 while passing through between the protruding patterns 54 of the electroform 50. As the resin R, a thermosetting resin such as an epoxy resin or a silicone resin is preferably used, and particularly, a silicone resin is preferably used. In a case where the cavity 76 of the mold 70 is filled with the resin R, the resin R is then heated and the resin R is cured.

Among silicone resins, there are one-component thermosetting silicone materials, two-component mixed curable silicone materials, UV-curable silicone materials, and the like. Most medical silicone resins are two-component mixed curable silicone materials. In the present embodiment, the resin R is obtained by mixing two components and mixing a color material (colorant) therein in an amount of 5 mass% or less with respect to the mass of a transparent silicone material. Details of the colorant will be described later.

As illustrated in Fig. 8, in order to release the cured resin R from the electroform 50, the first mold 72 and the second mold 74 clamped are opened. During the opening, the first mold 72 and the second mold 74 are moved away from each other. As illustrated in Fig. 8, the second mold 74 has the depression 84 for forming the cavity 76. The cured resin R is a mold 124 on which a plurality of recessed patterns 130 before releasing are formed.

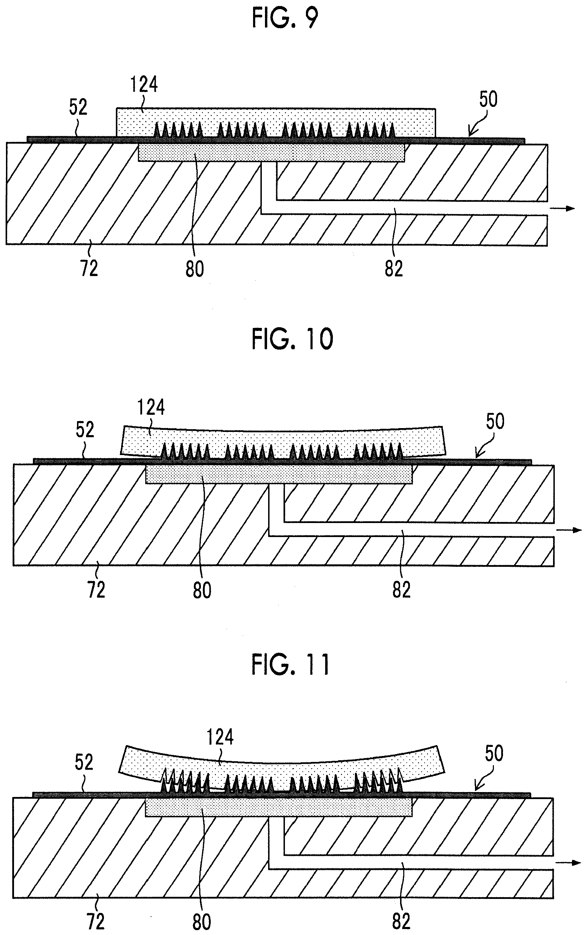

As illustrated in Fig. 9, the first mold 72 is separated from the second mold 74 and is moved to a stage for releasing the mold 124 from the electroform 50. In the present embodiment, since the second mold 74 having the depression 84 is separated from the mold 124, the mold 124 excluding the surface being in contact with the electroform 50 fixed to the first mold 72 is exposed. Therefore, in a case where the mold 124 is released from the electroform 50, it is possible to easily release the mold 124 using the exposed surface of the mold 124.

As illustrated in Fig. 10, the peripheral portion of the mold 124 is first separated from the electroform 50. The peripheral portion of the mold 124 may include at least two opposing sides in a case where the mold 124 is viewed in a plan view, and may include all of the four sides. The peripheral portion means a region from the outer periphery of the mold 124 to the recessed pattern 130.

As illustrated in Fig. 11, the peripheral portion of the mold 124 is gradually separated from the electroform 50. In a case where the mold 124 is made of a silicone resin, since the mold 124 has elasticity, the mold 124 enters a stretched state (elastically deforms) as the peripheral portion of the mold 124 is gradually separated. As the peripheral portion of the mold 124 is further separated from the electroform 50, the elastically deformed mold 124 tries to return to its original shape, so that the mold 124 contracts. By using the contraction force of the mold 124, the mold 124 is released from the electroform 50. By using the contraction force of the mold 124 as the releasing force, no excessive force is applied between the mold 124 and the protruding patterns 54 of the electroform 50, so that it is possible to suppress failure in releasing.

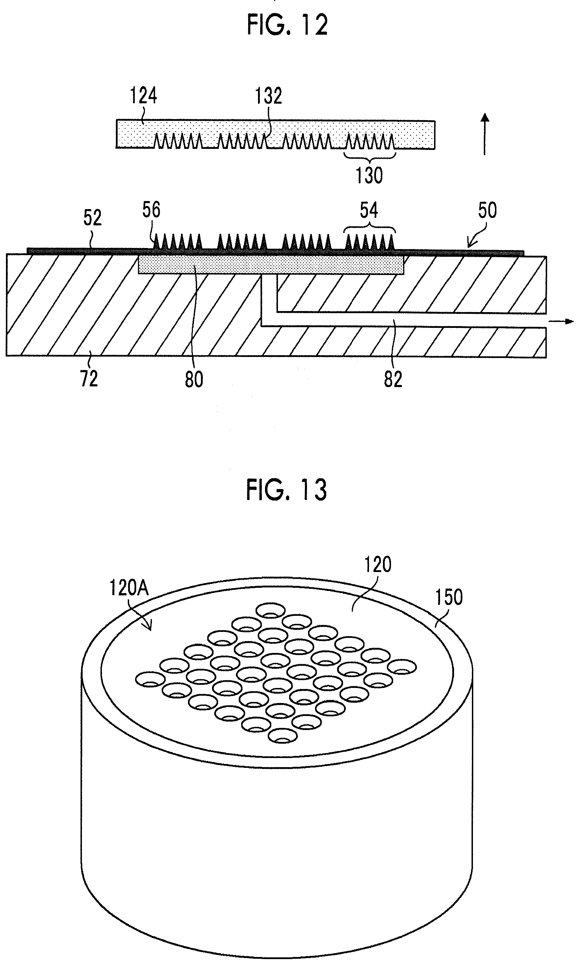

As illustrated in Fig. 12, finally, the mold 124 and the protruding patterns 54 of the electroform 50 are completely released from each other, and the mold 124 having the recessed patterns 130 is produced. The mold 124 is in a state in which a plurality of the molds 120 illustrated in Fig. 2 are connected.

In a case where the mold 124 is repeatedly produced from the electroform 50, the protruding patterns 54 are gradually damaged, and after use about 1000 to 10,000 times, it is necessary to replace the electroform 50 with a new electroform 50. In the present embodiment, by stopping the driving of the vacuum pump (not illustrated) and reducing the adsorption force of the adsorption plate 80, the electroform 50 can be replaced within a short period of time.

As a method of separating the peripheral portion of the mold 124 from the electroform 50, there is a method of suctioning the peripheral portion of the mold 124 in the exposed surface opposite to the surface on which the recessed patterns 130 are formed with suctioning means and separating the suctioning means from the electroform 50 while suctioning the peripheral portion.

It is desirable that the mold 124 (mold 120) produced in this manner has excellent gas permeability.

### <Method for Manufacturing Percutaneous Absorption Sheet>

Fig. 13 is a perspective view of a transporting jig 150 on which the mold 120 is mounted. In the manufacturing of the percutaneous absorption sheet, the mold 120 is mounted on the transporting jig 150 and handled. The transporting jig 150 is made of a plastic such as polypropylene. The transporting jig 150 supports the mold 120 in a state in which the front surface 120A of the mold 120 faces upward in a Z direction, which is a vertical direction, and the sheet portion 102 of the mold 120 is parallel to an XY plane, which is a horizontal plane.

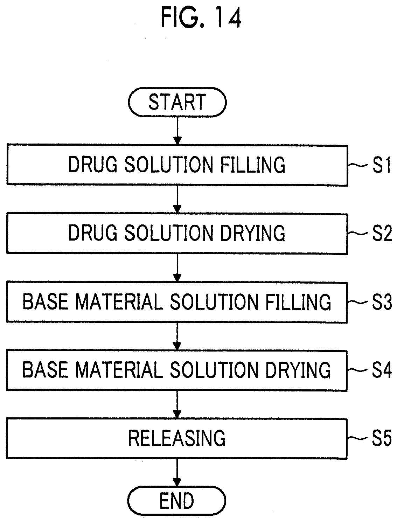

Fig. 14 is a flowchart showing each step of a method for manufacturing the percutaneous absorption sheet 100. The method for manufacturing the percutaneous absorption sheet 100 includes a drug solution filling step (step S1) of filling the needle-like recessed portion 132 of the mold 120 with a drug solution, a drug solution drying step (step S2) of drying the filled drug solution, a base material solution filling step (step S3) of filling the needle-like recessed portion 132 with a base material solution, a base material solution drying step (step S4) of drying the filled base material solution, and a releasing step (step S5) of releasing the formed percutaneous absorption sheet 100 from the mold 120.

### [Drug Solution Filling Step (Step S1)]

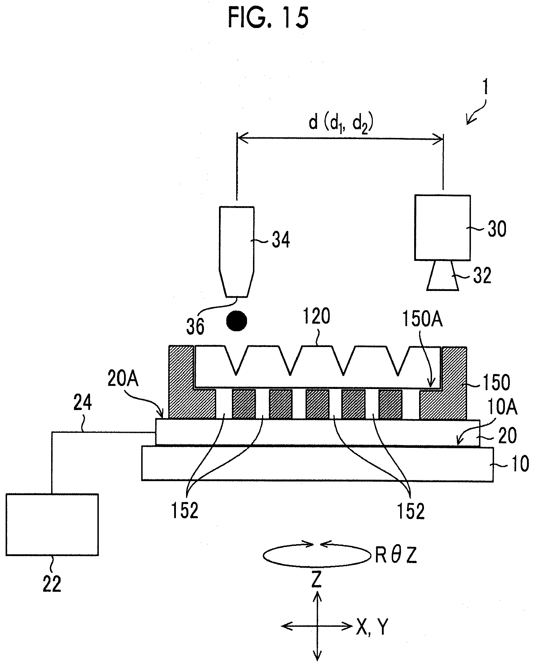

In the drug solution filling step, a liquid droplet of the drug solution is ejected from a nozzle 36 (see Fig. 15) of a drug solution ejection head 34 toward the needle-like recessed portion 132 of the mold 120, and the mold 120 is suctioned by a suction pump 22 (see Fig. 15). Details of the drug solution filling step will be described later.

### [Drug Solution Drying Step (Step S2)]

In the drug solution drying step, for example, drying is performed by blowing air to the drug solution filling the needle-like recessed portion 132. The environment around the mold 120 may be reduced in pressure.

### [Base Material Solution Filling Step (Step S3)]

In the base material solution filling step, the needle-like recessed portion 132 is filled with the base material solution. The base material solution is a drug-free polymer solution, and as water-soluble polymer substance forming the polymer solution, it is preferable to use a water-soluble polymer substance such as chondroitin sulfate, hydroxyethyl starch, or dextran.

Examples of a method for filling the needle-like recessed portion 132 with the base material solution include application using a spin coater.

### [Base Material Solution Drying Step (Step S4)]

In the base material solution drying step, as in the drug solution drying step, drying is performed by blowing air to the base material solution filling the needle-like recessed portion 132.

### [Releasing Step (Step S5)]

In the releasing step, the sheet (percutaneous absorption sheet 100) formed by drying the drug solution and the base material solution is released from the mold 120.

### <Drug Solution Filling Apparatus>

Fig. 15 is a schematic configuration diagram of a drug solution filling apparatus 1 (an example of an apparatus for manufacturing a percutaneous absorption sheet) used in the drug solution filling step. The drug solution filling apparatus 1 includes an XYZ stage 10, an adsorption plate 20, the suction pump 22, an alignment camera 30, the drug solution ejection head 34, and the like.

The XYZ stage 10 (an example of a positioning unit) has a placement surface 10A parallel to an XY plane. The XYZ stage 10 is provided so as to be movable by a motor (not illustrated) in an X direction and a Y direction orthogonal to the X direction, which are two directions parallel to the XY plane. Furthermore, the XYZ stage 10 is provided so as to be movable also in a Z direction and an RθZ direction, which is a rotation direction with the direction parallel to the Z direction as the axis.

The adsorption plate 20 is fixed to the placement surface 10A of the XYZ stage 10. The adsorption plate 20 has a placement surface 20A parallel to the XY plane. The placement surface 20A is provided with a plurality of adsorption holes (not illustrated). The adsorption plate 20 may be made of a porous member.

The suction pump 22 is connected to the adsorption plate 20 via a suction pipe 24. By driving the suction pump 22, air can be suctioned from the plurality of adsorption holes (not illustrated) of the placement surface 20A of the adsorption plate 20.

The transporting jig 150 is placed on the placement surface 20A of the adsorption plate 20. In the transporting jig 150, the mold 120 is mounted on a placement surface 150A. Accordingly, the mold 120 can move in each direction as the XYZ stage 10 moves in the X direction, the Y direction, the Z direction, and the RθZ direction.

A plurality of adsorption holes 152 pass through the placement surface 150A of the transporting jig 150. By driving the suction pump 22, the rear surface 120B of the mold 120 is suctioned via the plurality of adsorption holes (not illustrated) of the placement surface 20A of the adsorption plate 20 and the plurality of adsorption holes 152 of the transporting jig 150.

The alignment camera 30 comprises, in addition to an imaging lens 32, an imaging element (not illustrated), an analog-to-digital converter, and an image processing circuit.

The imaging lens 32 is a lens group comprising a zoom lens, a focus lens, and the like, and causes incidence ray from a subject to be incident onto the imaging element.

The imaging element is a charge coupled device (CCD) type imaging element or a complementary metal oxide semiconductor (CMOS) type imaging element in which a large number of light-receiving elements are two-dimensionally arranged on an imaging surface (not illustrated). The imaging element is disposed in a rear stage of an optical path of the incidence ray of the imaging lens 32.

The imaging lens 32 forms an image of the incidence ray on an imaging surface of the imaging element. The imaging element outputs an analog imaging signal corresponding to the amount of received light. This imaging signal is converted into a digital signal by the analog-to-digital converter, and then generated into an image signal by the image processing circuit.

The alignment camera 30 is disposed above the XYZ stage 10 in the Z direction, and the imaging lens 32 is directed downward in the Z direction. Accordingly, the alignment camera 30 can image the mold 120 placed on the XYZ stage 10.

The drug solution ejection head 34 is disposed at a position above the XYZ stage 10 in the Z direction and separated from the alignment camera 30 by a distance d on the XY plane including a distance d₁ in the X direction and a distance d₂ in the Y direction. The drug solution ejection head 34 includes the nozzle 36 (an example of a drug solution ejection nozzle) that ejects liquid droplets of the drug solution in a first direction. Here, the nozzle 36 is directed downward in the Z direction, and the first direction is a downward direction in the Z direction. The drug solution ejection head 34 illustrated in Fig. 15 includes one nozzle 36, but may include a plurality of nozzles 36.

As the drug solution ejection head 34, for example, an ink jet head such as a solenoid type ink jet head or a piezoelectric ink jet head can be used. The amount of one liquid droplet ejected from the nozzle 36 is about 1 to 150 nL.

The drug solution ejected from the nozzle 36 flies downward in the Z direction and lands on an object (in this case, the mold 120). Therefore, the position of the nozzle 36 on the XY plane and the position on the XY plane where the drug solution lands are the same.

The drug solution contains a drug stock solution, a sugar, an additive, and the like as the drug. Moreover, the drug solution contains water, ethanol, or the like as a solvent.

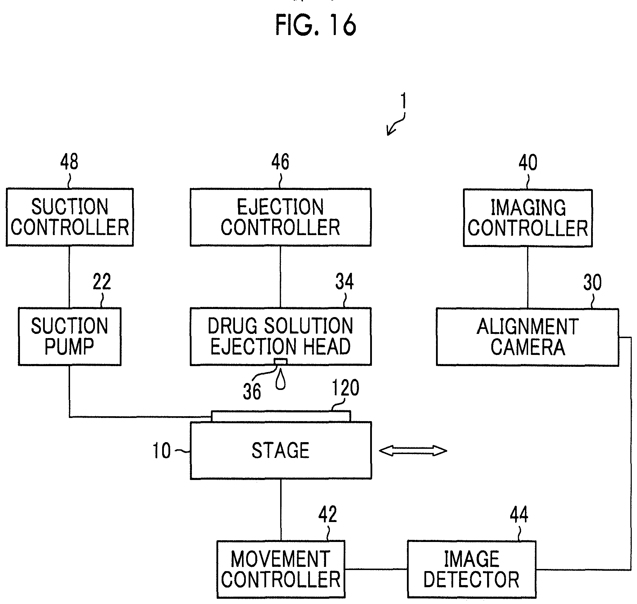

Fig. 16 is a block diagram illustrating an electrical configuration of the drug solution filling apparatus 1. The drug solution filling apparatus 1 comprises an imaging controller 40, a movement controller 42, an image detector 44, an ejection controller 46, a suction controller 48, and the like.

The imaging controller 40 causes the alignment camera 30 to take an image.

The movement controller 42 controls a relative movement between the mold 120 placed on the XYZ stage 10 and the drug solution ejection head 34. Here, the mold 120 is moved by driving the XYZ stage 10, but the drug solution ejection head 34 may be moved, or both the mold 120 and the drug solution ejection head 34 may be moved.

The image detector 44 detects the position of the mold 120 based on the image of the mold 120 imaged by the alignment camera 30. In the present embodiment, the position of the needle-like recessed portion 132 is detected by recognizing the needle-like recessed portion 132 from the image of the mold 120.

The ejection controller 46 controls the timing of ejecting the drug solution from the nozzle 36, and the liquid droplet amount of the drug solution to be ejected, by controlling the drug solution ejection head 34.

The suction controller 48 controls the presence or absence of suction by the suction pump 22.

### <Drug Solution Filling Step>

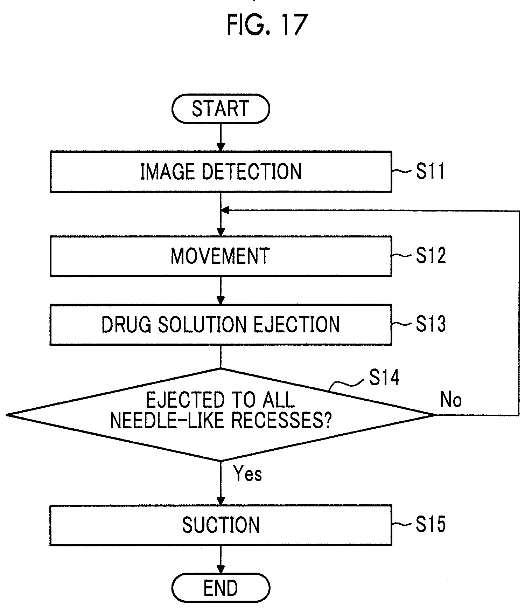

Fig. 17 is a flowchart showing each step included in the drug solution filling step. The drug solution filling step includes an image detection step (step S11), a movement step (step S12), a drug solution ejection step (step S13), a determination step (step S14), and a suction step (step S15).

### [Image Detection Step (Step S11)]

First, the transporting jig 150 on which the mold 120 is mounted is placed on the placement surface 20A of the adsorption plate 20.

The movement controller 42 controls the XYZ stage 10 to move the mold 120 within the angle of view of the image taken by the alignment camera 30. The imaging controller 40 controls the alignment camera 30 to take an image of the mold 120. The image detector 44 detects the position of each needle-like recessed portion 132 by analyzing the image of the mold 120 taken by the alignment camera 30.

For example, the needle-like recessed portion 132 of the mold 120 is moved to the center within the angle of view of the taken image of the alignment camera 30 by the XYZ stage 10, and the XY plane coordinates (X,Y) of the XYZ stage 10 at this point are calculated. By performing this for all the needle-like recessed portions 132, the positions of all the needle-like recessed portions 132 can be detected.

In the image of the mold 120 taken by the alignment camera 30, the flat portion 122 has a relatively bright brightness, and the needle-like recessed portion 132 has a relatively dark brightness. By using this contrast, the needle-like recessed portion 132 can be moved to the center within the angle of view of the taken image of the alignment camera 30.

Instead of moving all the needle-like recessed portions 132 to the center within the angle of view of the image taken by the alignment camera 30, only the XY plane coordinates (X,Y) of three to five needle-like recessed portions 132 may be detected and the direction (rotation) of the mold 120 in the XY plane from the coordinates and the expansion and contraction of the mold 120 in the XY plane may be analyzed to detect the positions of the other needle-like recessed portions 132.

Alternatively, the mold 120 may be provided with a plurality of alignment marks, and the XY plane coordinates (X,Y) of the needle-like recessed portion 132 may be detected by reading the alignment marks.

Furthermore, the position (height) of the mold 120 in the Z direction may be adjusted by measuring the distance between the needle-like recessed portion 132 or the alignment mark and the alignment camera 30. The distance between the nozzle 36 and the mold 120 is preferably adjusted to be 0.5 mm to 5 mm, and preferably 1 mm to 2 mm.

### [Movement Step (Step S12)]

The movement controller 42 controls the XYZ stage 10 based on the detection result of the image detector 44 to move and mechanically position the mold 120 in the X direction and the Y direction so as to cause the position of the nozzle 36 of the drug solution ejection head 34 on the XY plane and the position of the needle-like recessed portion 132 on the XY plane to coincide with each other. That is, the position of the nozzle 36 and the position of the needle-like recessed portion 132 are caused to coincide with each other in a plan view in the direction (Z direction) parallel to the ejection direction of the drug solution from the nozzle 36.

Coordinates (X + d₁,Y + d₂) obtained by adding the distance d₁ in the X direction between the alignment camera 30 and the nozzle 36 of the drug solution ejection head 34 and the distance d₂ in the Y direction to the coordinates (X,Y) of the needle-like recessed portion 132 calculated in step S11 are the coordinates of the nozzle 36. The movement controller 42 moves the XYZ stage 10 to the coordinates.

### [Drug Solution Ejection Step (Step S13)]

The ejection controller 46 controls the drug solution ejection head 34 to eject the drug solution from the nozzle 36. The ejected drug solution lands on the needle-like recessed portion 132. Here, one droplet of the drug solution is ejected from the nozzle 36 to one needle-like recessed portion 132 and is caused to land on the needle-like recessed portion 132. Alternatively, a plurality of droplets of the drug solution may be caused to land on one needle-like recessed portion 132.

The drug solution that lands on the needle-like recessed portion 132 needs to block the needle-like recessed portion 132, that is, be into contact with the entire circumferential surface of the needle-like recessed portion 132. In a case where the landed drug solution does not block the needle-like recessed portion 132, the landed drug solution cannot fill the distal end of the tapered shape of the distal end recessed portion 134 in the suction step, which will be described later. Therefore, in order to cause the position of the nozzle 36 and the position of the needle-like recessed portion 132 to coincide with each other in the movement step, accurate positioning is required.

### [Determination Step (Step S14)]

The ejection controller 46 determines whether or not the drug solution has been ejected to land on all the needle-like recessed portions 132 of the mold 120.

In a case where it is determined that there is a needle-like recessed portion 132 on which the drug solution does not land, the process returns to step S12 and the same processing is performed. That is, the position on the XY plane of the needle-like recessed portion 132 to which the drug solution has not been ejected and the position on the XY plane of the nozzle 36 are caused to coincide with each other (step S12), and the drug solution is ejected from the nozzle 36 to land on the needle-like recessed portion 132 (step S13).

In a case where it is determined that the drug solution has landed on all the needle-like recessed portions 132, the process proceeds to step S15.

### [Suction Step (Step S15)]

The suction controller 48 drives the suction pump 22 to suction the rear surface 120B of the mold 120. By this suction, the drug solution that has landed on the needle-like recessed portion 132 fills the distal end of the tapered shape of the distal end recess 134.

As above, the drug solution filling step is finished. The drug solution ejection step and the suction step may be performed at the same time. That is, the drug solution may be ejected from the nozzle 36 while suctioning is performed by the suction pump 22.

Here, the distance d₁ and the distance d₂ are treated as known values, but in a case where the distances are unknown, the distances can be obtained as follows.

A dummy mold that is not provided with the needle-like recessed portions 132 is mounted on the transporting jig 150 and placed on the placement surface 20A of the adsorption plate 20. The drug solution is ejected from the nozzle 36 to the dummy mold so as to land on the dummy mold.

Next, the XYZ stage 10 is moved in the X direction and the Y direction so that the landed drug solution is disposed at the center of the angle of view of the taken image of the alignment camera 30. Here, the amount of movement of the XYZ stage 10 in the X direction is the distance d₁, and the amount of movement thereof in the Y direction is the distance d₂.

### <Color of Mold>

In a case where the mold 120 is transparent, the adsorption holes 152 of the transporting jig 150 are seen through the upper surface of the mold 120, and the adsorption holes 152 are reflected in the image of the mold 120 taken by the alignment camera 30. The reflection of the adsorption holes 152 becomes an obstacle in a case where the image detector 44 recognizes the position of the needle-like recessed portion 132 from the image, and the accuracy of position detection decreases. In order to prevent the adsorption holes 152 from being reflected, the mold 120 is preferably opaque. By using the opaque mold 120, the needle-like recessed portions 132 in the image can be properly recognized.

### Examples

Whether or not image recognition of the needle-like recessed portion of the mold mounted on the transporting jig 150 was possible was evaluated. In the present embodiment, in order to manufacture the opaque mold 120, a colorant was mixed in advance in the resin R supplied to the cavity 76.

A silicone material (SILASTIC MDX4-4210 manufactured by Dow Corning) was used as the primary material of the mold.

In addition, the following were used as the colorant for each color.
White: NuSil MED-4900-1
Black: NuSil MED-4900-2
Red: NuSil MED-4900-4
Yellow: NuSil MED-4900-5
Blue: NuSil MED-4900-8

The material of the mold was a resin R illustrated in Fig. 7, which was obtained by mixing the primary agent:hardener: colorant in a mass ratio of 10:1:X. The amount of the colorant was 5 mass% or less, and more specifically 0.4 mass% to 3.6 mass%.

Whether or not image recognition was possible was determined by the following procedure using an image dimension measurement system IM-6015 manufactured by Keyence Corporation.
(1) Of the plurality of needle-like recessed portions formed in the mold, five needle-like recessed portions designated in advance were subjected to edge extraction to detect respective images thereof as circles, and each of the center coordinates of the needle-like recessed portions subjected to the image detection was calculated.
(2) In (1), a case where the images of all the five designated needle-like recessed portions were detected as circles was evaluated as image detection possible, and other cases were evaluated as image detection impossible.
(3) The direction of the mold was changed (the mold was rotated about an axis parallel to the optical axis of the imaging optical system), and whether or not image detection was possible was checked for 10 different directions of the mold.

As optical settings, reflected light was used instead of telecentric transmitted light. The amount of reflected light was kept constant at 100% for detection (integration).

Sample A was a transparent mold in which no colorant was used. Sample B used a white colorant. Sample C used a red colorant. Sample D used a yellow colorant. Sample E used a blue colorant. Sample F used a black colorant.

As the evaluation criteria for determining whether or not image recognition was possible, a case where 10 times of image detection were possible for 10 different directions of the mold was evaluated as "best", a case where 8 or more times of image detection were possible was evaluated as "good", a case where 6 or more times of image detection were possible was evaluated as "possible", and a case where 5 or less times of image detection were performed was evaluated as "impossible".

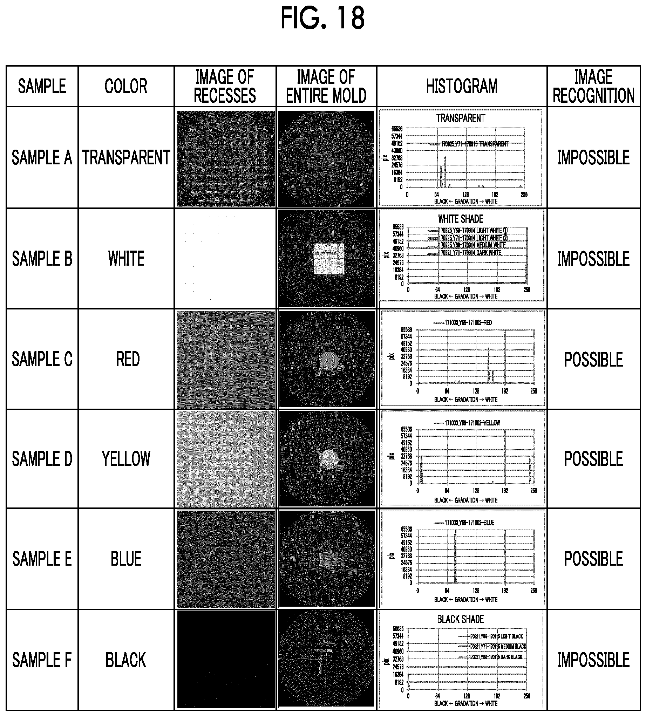

Fig. 18 shows taken images of the needle-like recessed portions of Samples A to F, taken images of the entire mold, histograms of the brightness of the taken images of the needle-like recessed portions, and whether or not image recognition was possible.

As shown in Fig. 18, in Sample A, the image recognition of the needle-like recessed portions was evaluated as "impossible" due to the influence of the plurality of adsorption holes 152 of the transporting jig 150. In Sample B, the image recognition of the needle-like recessed portions was evaluated as "possible", but the amount of reflected light was large and the image of the entire mold exceeded a threshold value.

In Samples C, D, and E, the image recognition was evaluated as "possible". In Sample F, the needle-like recessed portions were not reflected, and the image recognition was evaluated as "impossible".

As described above, it could be seen that it was difficult to produce an optimal mold for image recognition simply by mixing the monochromatic colorants.

Samples G to K were gray molds using a white colorant and a black colorant. In Sample G, Sample H, Sample I, Sample J, and Sample K, the mass ratio between the white colorant and the black colorant was set to 3:1, 4:1, 5:1, 7:1, and 10:1.

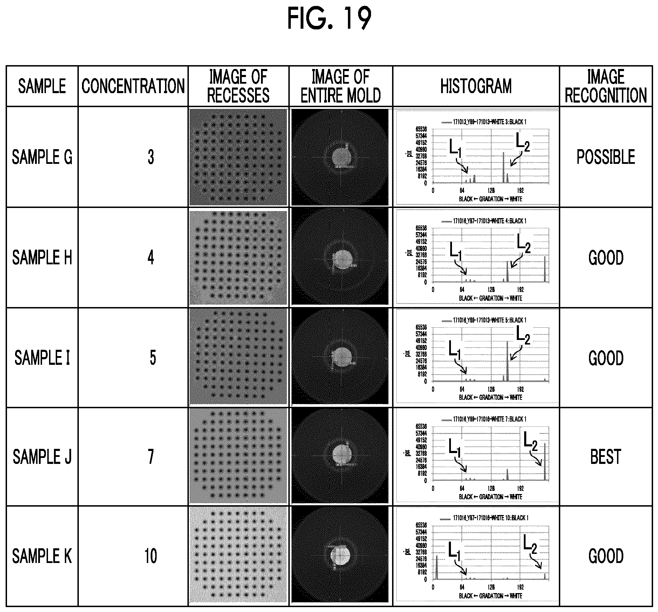

Fig. 19 shows taken images of the needle-like recessed portions of Samples G to K, taken images of the entire mold, histograms of the brightness of the taken images of the needle-like recessed portions, and whether or not image recognition was possible. As shown in Fig. 19, it could be seen that the taken images of the entire mold were taken with high brightness in the order of Sample G, Sample H, Sample I, Sample J, and Sample K.

In Sample G, the image recognition of the needle-like recessed portions was evaluated as "possible". The brightness L₁ corresponding to the needle-like recessed portion 132 was observed as a set of three brightnesses due to reflection from a plurality of different positions from the distal end recessed portion 134 and the cup portion 136. The brightness L₂ corresponding to the base portion (corresponding to the flat portion 122 in Fig. 2) appeared on the brighter side than the brightness L₁. In the histogram of Sample G, the brightness L₁ corresponding to the needle-like recessed portion and the brightness L₂ corresponding to the base portion each had a constant appearance frequency, and it could be seen that the needle-like recessed portion and the base portion could be distinguished from each other.

In Sample H and Sample I, the image recognition was evaluated as "good". As shown in the histograms, the difference between the brightness L₁ corresponding to the needle-like recessed portion and the brightness L₂ corresponding to the base portion was larger than that of Sample G, and it could be seen that the needle-like recessed portion and the base portion could be easily distinguished from each other.

In Sample J, the image recognition was evaluated as "best". As shown in the histogram, the difference between the brightness L₁ corresponding to the needle-like recessed portion and the brightness L₂ corresponding to the base portion was larger than those of Sample H and Sample I, and it could be seen that the needle-like recessed portion and the base portion could be more easily distinguished from each other.

In Sample K, the image recognition was evaluated as "good". As shown in the histogram, although the difference between the brightness L₁ corresponding to the needle-like recessed portion and the brightness L₂ corresponding to the base portion was the same as that of Sample J, the appearance frequency was low, and distinguishing between the needle-like recessed portion and the base portion was inferior to that of Sample J.

As described above, by mixing the white colorant and the black colorant in the transparent resin, a mold suitable for image recognition could be produced. Here, it could be seen that the mass ratio between the white colorant and the black colorant was preferably 3:1 to 10:1, and a mass ratio of 7:1 between the white colorant and the black colorant was most suitable for image recognition.

### <Definition of Gray Color>

The gray color of the mold is defined.

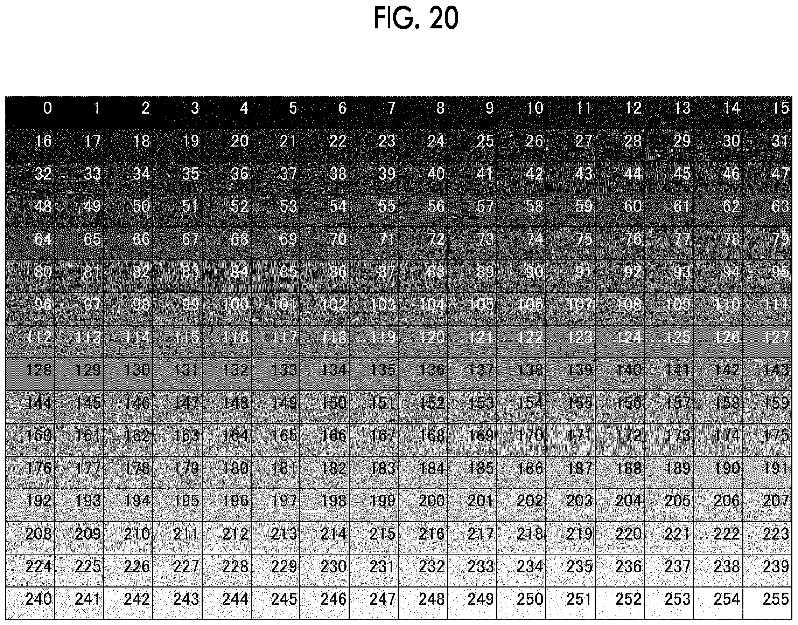

First, a sample showing an 8-bit grayscale (256 gradations) is created. Fig. 20 is an example of the 8-bit grayscale sample. In Fig. 20, the numerical value described in each cell is a brightness value that represents a brightness from the lowest brightness (0) to the highest brightness (256) in 256 levels in the HSL (Hue Saturation Lightness) color system (color space), and each cell is filled with the gradation of the corresponding brightness value. Fig. 20 shows a sample in a case where a saturation value that represents a saturation from the lowest saturation (0) to the highest saturation (256) in 256 levels is 0.

That is, in a case where each brightness value shown in Fig. 20 is represented by the 8-bit HSL color system, the brightness value can be represented as (hue, saturation, brightness) = (X, 0, 0 to 255). In this manner, the hue can use an any value X of 0 to 255.

Here, a range in which a saturation value is 0 to 25 (a 10% range on the low saturation side), and a range in which a brightness value L is 25 to 230 (a range excluding 10% on the brightness side and 10% on the high brightness side) are defined as a gray color.

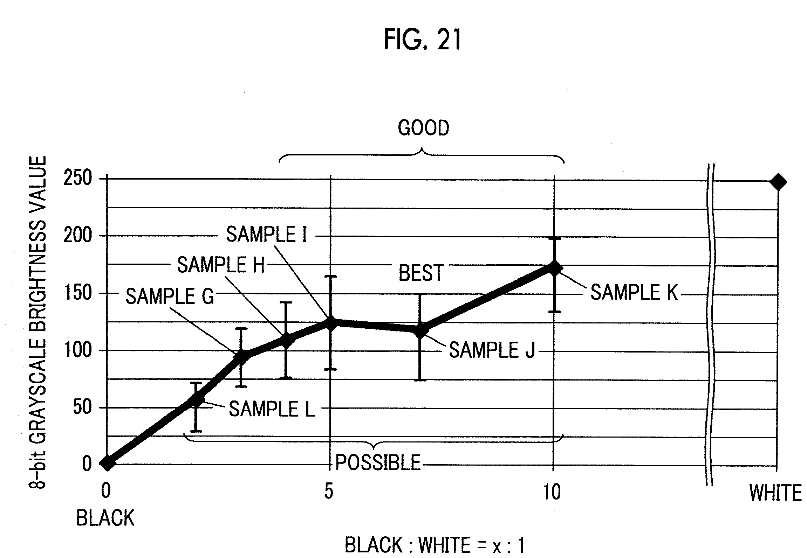

For Samples G to K shown in Fig. 19 and Sample L in which the mass ratio between the white colorant and the black colorant was 2:1 and the image recognition was evaluated as "possible", compared to the sample shown in the 8-bit grayscale shown in Fig. 20, the gray color of the brightness value to which the gray color of each sample corresponds was evaluated. The number of evaluators is five. The results are shown in Fig. 21.

As shown in Fig. 21, the evaluation values (brightness values) of the five evaluators were 70 to 120 for Sample G, 75 to 140 for Sample H, 80 to 165 for Sample I, 75 to 150 for Sample J, 135 to 200 for Sample K, and 30 to 70 for Sample L.

From this result, it could be seen that the range in which the image recognition was evaluated as "possible" or higher was a gray color in a brightness value range of 30 to 200, the range in which the image recognition was evaluated as "good" or higher was a gray color in a brightness value range of 75 to 200, and the range in which the image recognition was evaluated as "best" or higher was a gray color in a brightness value range of 75 to 150 or less.

In addition, in s saturation value range of 1 to 25, the same sample as that of Fig. 20 was created and the same evaluation was performed. That is, the same evaluation was performed on the sample of (H, S, L) = (X, 1 to 25, 0 to 255).

As a result, there was no significant difference from the evaluation result shown in Fig. 21.

As described above, it could be seen that by using a mold having a gray color in which the brightness value in a case where the brightness in the HSL color system was represented in 256 levels was in a range of 30 or more and 200 or less, the image recognition could be appropriately performed. It could also be seen that a mold having a gray color in which the brightness value is in a range of 75 or more and 200 or less is more preferable, and a mold having a gray color in which the brightness value is in a range of 75 or more and 150 or less is even more preferable. In this case, gray colors in which the saturation value in a case where the saturation in the HSL color system was represented in 256 levels was in a range of 0 or more and 25 or less were preferable. In addition, in these gray colors, an any value can be used for the hue in the HSL color system.

In this example, the amount of the colorant was set to 0.4 mass% to 3.6 mass% with respect to the two-component mixed silicone material, but it could be confirmed that there was no influence on the image recognition accuracy in this range.

Furthermore, in this example, SILASTIC MDX4-4210 manufactured by Dow Corning was used as the primary material of the mold, but other silicone materials such as NuSil MED-6019 manufactured by Dow Corning can be used. While SILASTIC MDX4-4210 manufactured by Dow Corning is a silicone material that obtained by mixing a primary agent and a hardener in a ratio of 10:1 and curing the mixture, NuSil MED-6019 is a silicone material that is obtained by mixing an A agent and a B agent in a ratio of 1:1 and curing the mixture.

The order in which the colorant is mixed in the transparent silicone material is not particularly important.

The white colorant may be mixed in the A agent, the black colorant may be mixed in the B agent, and finally the A agent containing the white colorant and the B agent containing the black colorant may be mixed with each other. The black colorant may be mixed in the A agent and the white colorant may be mixed in the B agent. Alternatively, a color material which is a gray agent (gray colorant) obtained by appropriately mixing the white colorant and the black colorant is manufactured by a material manufacturer, and a mold manufacturer may mix the A agent, the B agent, and the gray agent together.

### <Others>

The technical scope of the present invention is not limited to the scope described in the above embodiment. The configurations and the like in the embodiments can be appropriately combined between the embodiments without departing from the gist of the present invention.

### Explanation of References

1: drug solution filling apparatus
10: XYZ stage
10A: placement surface
20: adsorption plate
20A: placement surface
22: suction pump
24: suction pipe
30 alignment camera
32: imaging lens
34: drug solution ejection head
36: nozzle
40: imaging controller
42: movement controller
44: image detector
46: ejection controller
48: suction controller
50: electroform
52: first surface
54: protruding pattern
56: needle-like protrusion
58: second surface
70: mold
72: first mold
74: second mold
76: cavity
78: flat surface
80: adsorption plate
82: suction pipe
84: depression
86: gate
88: injection molding machine
100: percutaneous absorption sheet
100A: front surface
100B: rear surface
102: sheet portion
110: protruding pattern
112: needle-like protrusion
114: needle portion
116: frustum portion
120: mold
120A: front surface
120B: rear surface
122: flat portion
124: mold
130: recessed pattern
132: needle-like recessed portion
134: distal end recessed portion
136: cup portion
150: transporting jig
150A: placement surface
152: adsorption hole
S1 to S5: each step of method for manufacturing percutaneous absorption sheet
S11 to S15: each step included in drug solution filling step

## Claims

1. A mold for manufacturing a percutaneous absorption sheet in which a plurality of needle-like recessed portions are disposed,
wherein the mold has a gray color in which a brightness value in a case where a brightness in an HSL (Hue Saturation Lightness) color space is represented in 256 levels is in a range of 30 or more and 200 or less.

2. The mold for manufacturing a percutaneous absorption sheet according to claim 1,
wherein the mold has a gray color in which the brightness value is in a range of 75 or more.

3. The mold for manufacturing a percutaneous absorption sheet according to claim 2,
wherein the mold has a gray color in which the brightness value is in a range of 150 or less.

4. The mold for manufacturing a percutaneous absorption sheet according to any one of claims 1 to 3,
wherein the mold has a gray color in which a saturation value in a case where a saturation in an HSL color space is represented in 256 levels is in a range of 0 or more and 25 or less.

5. The mold for manufacturing a percutaneous absorption sheet according to any one of claims 1 to 4,
wherein the mold includes a transparent resin, a white colorant, and a black colorant.

6. The mold for manufacturing a percutaneous absorption sheet according to claim 5,
wherein a mass ratio between the white colorant and the black colorant is 3:1 to 10:1.

7. The mold for manufacturing a percutaneous absorption sheet according to claim 6,
wherein the mass ratio between the white colorant and the black colorant is 7:1.

8. The mold for manufacturing a percutaneous absorption sheet according to any one of claims 5 to 7,
wherein an amount of the white colorant and the black colorant is 5 mass% or less.

9. The mold for manufacturing a percutaneous absorption sheet according to any one of claims 5 to 8,
wherein the resin is a silicone resin.

10. A method for manufacturing a percutaneous absorption sheet having a needle-like protrusion, the method comprising:
an image detection step of detecting the mold for manufacturing a percutaneous absorption sheet from an image obtained by imaging the mold for manufacturing a percutaneous absorption sheet according to any one of claims 1 to 9;
a positioning step of mechanically positioning at least one of a drug solution ejection nozzle or the mold for manufacturing a percutaneous absorption sheet based on a detection result of the image detection step; and
an ejection step of ejecting a drug solution from the drug solution ejection nozzle toward a needle-like recessed portion of the mold for manufacturing a percutaneous absorption sheet.

11. The method for manufacturing a percutaneous absorption sheet having a needle-like protrusion according to claim 10, further comprising:
a suction step of suctioning the mold for manufacturing a percutaneous absorption sheet to fill the needle-like recessed portion with the drug solution.

12. The method for manufacturing a percutaneous absorption sheet having a needle-like protrusion according to claim 11,
wherein the mold for manufacturing a percutaneous absorption sheet is placed on a transporting jig, and
the transporting jig is provided with an adsorption hole for suctioning the mold for manufacturing a percutaneous absorption sheet in the suction step.

13. The method for manufacturing a percutaneous absorption sheet having a needle-like protrusion according to any one of claims 10 to 12, further comprising:
a drying step of drying the drug solution filling the needle-like recessed portion.

14. An apparatus for manufacturing a percutaneous absorption sheet having a needle-like protrusion, the apparatus comprising:
a camera that images the mold for manufacturing a percutaneous absorption sheet according to any one of claims 1 to 9;
an image detector that detects the mold for manufacturing a percutaneous absorption sheet from an image obtained by imaging the mold;
a drug solution ejection nozzle that ejects a drug solution toward a needle-like recessed portion of the mold for manufacturing a percutaneous absorption sheet; and
a positioning unit that mechanically positions at least one of the drug solution ejection nozzle or the mold for manufacturing a percutaneous absorption sheet based on a detection result of the image detector.
